# EUROPEAN PATENT APPLICATION

(11) **EP 2 950 097 A1**
(43) Date of publication of application: **02.12.2015**
(21) Application number: 14170202.7
(22) Date of filing: 28.05.2014
(51) Int. Cl.: G01N 33/574

(54) **Podoplanin as a biomarker of the activation of PI3K/mTOR signaling in human tumors**

(71) Applicant: Universitätsspital Basel, 4031 Basel (CH)
(72) Inventor: Wicki, Andreas, 4144 Arlesheim (CH); Ritschard, Reto, 4127 Birsfelden (CH); Prêtre, Vincent, 1564 Domdidier (CH)
(74) Representative: Latscha Schöllhorn Partner AG

(57) **Abstract**

The invention relates to the use of podoplanin as a biomarker of the activation of PI3K/mTor signaling in human tumors.

## Description

### Field of the invention

The invention relates to the use of podoplanin as a biomarker of the activation of PI3K/mTor signaling in human tumors.

### Background of the invention

Podoplanin, a small and highly glycosylated mucin-like transmembrane protein, plays a role in the development of organs and is present in several adult tissues, including brain, kidney, lungs, heart and lymphatic system. It is a widely used marker for lymphatic endothelial cells.

Podoplanin also promotes tumor cell spreading, migration and invasion in both mouse models of human cancer and human tumor samples. In human samples, podoplanin is coexpressed with E-cadherin in the invasive front of squamous cell carcinoma and, to a lesser extent, adenocarcinoma. Moreover, in a Rip1Tag2 mouse model, podoplanin promotes invasion of β-cell tumors, bypasses epithelial-to-mesenchymal transition and increases phosphorylation of ERM-proteins (Ezrin/Radixin/Moesin-proteins, A. Wicki et al., Cancer Cell, 2006, 9:261-72). While podoplanin expression patterns in many tissues have been well characterized, there is still little known about the physiological functions and possible interactions of this protein (A. Wicki, G. Christofori, Br J Cancer, 2007, 96:1-5).

### Summary of the invention

The invention relates to a method of determining whether, in a malignant human tumor, the PI3K and/or mTOR signaling pathway is activated, characterized in that tumor cells are analysed for the presence or absence of podoplanin, and the malignant tumor is classified as using the PI3K and/or mTOR signaling pathway if podoplanin is present.

Furthermore, the invention relates to podoplanin as a biomarker for PI3K/mTOR signaling in human malignant tumors.

Furthermore, the invention relates to a method of treating a malignant tumor in a human patient, wherein the presence or absence of podoplanin in the tumor is determined, and, if podoplanin is found, a drug active against PI3K and/or mTOR signaling pathways is administered.

### Brief description of the Figure

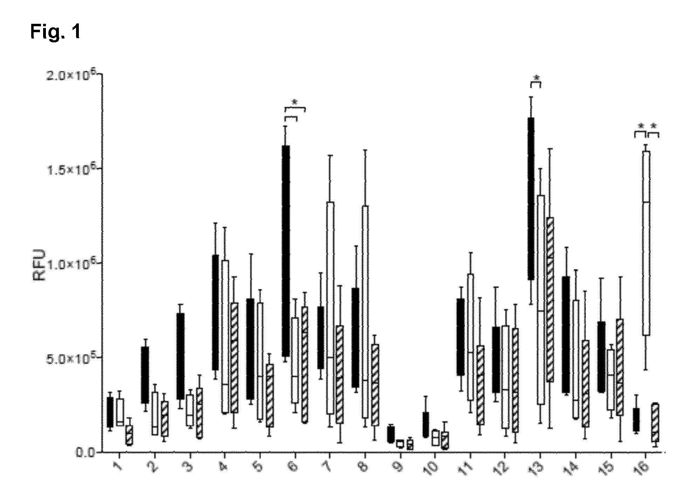

Figure 1: The Akt-mTOR pathway is activated in podoplanin-positive tumors in comparison to podoplanin-negative neoplasms. Therefore, podoplanin represents a predictive marker for response to drugs inhibiting the Akt-mTOR signaling pathway. RFU : Relative Fluorescent Units
   (1) Akt Thr308 (2) Akt Ser473 (3) S6 RibProt (4) AMPKa (5) PRAS40 (6) mTOR (7) GSK-3a (8) GSK-3ß (9) p70 S6 Kinase Thr389 (10) p70 S6 Kinase Thr421/Ser424 (11) Bad (12) RSK1 (13) PTEN (14) PDK1 (15) ERK1/2 (16) 4EBP1
   Akt = RAC-alpha serine/threonine-protein kinase
   S6 RibProt = 40S ribosomal protein S6
   AMPKa = 5'-AMP-activated protein kinase catalytic subunit alpha-1
   PRAS40 = Proline-rich AKT1 substrate 1
   mTOR = Serine/threonine-protein kinase mammalian target of rapamycin
   GSK-3a = Glycogen synthase kinase-3 alpha
   p70 S6 Kinase = p70 Ribosomal protein S6 kinase beta-1
   Bad = Bcl2 antagonist of cell death
   RSK1 = p90 Ribosomal protein S6 kinase alpha-1
   PTEN = Phosphatidylinositol 3,4,5-trisphosphate 3-phosphatase and dual-specificity protein phosphatase, phosphatase and tensin homolog
   PDK1 = 3-phosphoinositide-dependent protein kinase 1
   ERK1/2 = Extracellular signal-regulated kinase 1
   4EBP1 = Eukaryotic translation initiation factor 4E-binding protein 1
   Filled columns (■): Podoplanin positive squamous cell carcinoma
   Open columns (□): Podoplanin negative squamous cell carcinoma
   Striped columns ( 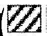 ): Podoplanin negative adenocarcinoma
   * p-value < 0.05

### Detailed description of the invention

The role of podoplanin in human tumors was analysed. The invasive tumor front was isolated by laser-capture microscopy and the samples analysed through PCR arrays (RNA) and phospho-protein analysis (as a sign of activation of specific intratumoral pathways). A total of 16 tumor samples were analysed: 6 podoplanin-positive (3 squamous cell carcinomas of the head-and-neck region and 3 of the lung) and 10 podoplanin negative (6 adenocarcinoma of the lung, 2 squamous cell carcinomas of the head-and-neck region and 2 of the lung).

Freshly-frozen serial biopsies from lung and head-and-neck cancer patients were sampled and analysed before and after oncological treatment. Tumor biopsies were freshly frozen in isopentane at -80°C, fixed 60 min in 4% paraformaldehyde, embedded in OCT (optimal temperature cutting compound, Tissue-Tek, Sakura Finetek, USA) medium, and cut into 8 µm sections on a cryostat. The plates were blocked for 1 hour at RT (room temperature) in PBS (phosphate buffered saline) + 4% BSA (bovine serum albumin) and incubated overnight at +4°C with primary antibody (from AngioBio, clone NZ-1). The plates were washed 3 x 5 min with PBS and incubated for 30 min at RT in a dark room with the secondary antibodies coupled with Alexa Fluor 488 or 555 (Invitrogen) and diluted 1:200 in PBS + 4% BSA. After washing, the fluorescent nuclear stain 4',6'-diamidino-2-phenylindole dihydrochloride (DAPI, Sigma Aldrich, Saint-Louis USA) was added and the plates incubated for 2 min at RT. Immunofluorescence microscopy for podoplanin was performed on a Olympus BX63 (Japan). The primary antibody we used for podoplanin-detection was from AngioBio, clone NZ-1 (Del Mar, CA, USA).

For podoplanin detection in routine experiments, tumors are fixed in 4% paraformaldehyde overnight at 4°C and embedded in paraffin. Immunohistochemistry on paraffin sections is done by quenching the slides in 3% H₂O₂ in PBS for 10 min, blocking in 5% goat serum for 1 h, incubation with primary antibody overnight at 4°C, incubation with secondary antibody for 30 min, and development with the ABC-kit of Vectastain (Vector Laboratories) and Sigma Fast 3,3'-diaminobenzidine tetrahydrochloride with metal enhancer (Sigma) or AEC-kit (Vector Laboratories). Incubation is performed with the same primary antibody (clone NZ-1).

For the standard quantitative RT-PCR, the whole slide total RNA was prepared using Trizol (Invitrogen), reverse transcribed with Maxima Reverse Transcriptase (Thermoscientific, USA), and transcripts were quantified by PCR using SYBR green PCR MasterMix (Applied Biosystems, Rotkreuz, Switzerland) and the primers. Human hypoxanthine phosphoribosyltransferase 1 (HPRT1) primers were used for normalization. PCR assays were performed in triplicate, and fold induction was calculated against control-treated cell lines using the comparative Ct method (ΔΔ*C*t). PCR primers were: PDPN 5'-AAATGTCGGGAAGGTACTCG-3' (SEQ ID NO:1) and 3-AGGGCACAGAGTCAGAAACG-5'(SEQ ID NO:2); HPRT1 5'-TGACCTTGATTTATTTTGCATACC-3' (SEQ ID NO:3) and 3'-CGAGCAAGACGTTCAGTCCT-5' (SEQ ID NO:4).

To be classified as podoplanin positive, a tumor has to (1) have an immunofluorescent-positive staining in the cortical region of the cell and be present in a connected group of more than 10 tumor cells, and (2) have a ΔΔCt ≥ 2 relative to HPRT1 (hypoxanthin-guanin-phosphoribosyltransferase-1) gene (ΔΔ*C*t = Δ*C*tₛₐₘₚₗₑ-Δ*C*t_{reference}) in the above described quantitative real-time PCR assay.

mRNA expression and the phospho-proteome of the isolated podoplanin-positive and podoplanin-negative tumor sections were analysed using PCR (Qiagen) and pathscan phospho-protein arrays (Cell Signaling Technology, USA). Podoplanin-positive tumors according to immunofluorescence have higher mRNA level of podoplanin than the podoplanin-negative tumors, see Table 1.

**Table 1**

| Group | N | PDPN Relative Expression (median and [range]) | t-test (Mann-Whitney) p-value |
|---|---|---|---|
| PDPN-positive | 34 | 4.457 [2.025 - 29.630] | < 0.0001 |
| PDPN-negative | 18 | 0.684 [0.082 - 1.443] | |

For mRNA analysis by PCR arrays, tumor biopsies were cut into 8 µm sections using a cryostat cutter and transferred onto LCM-specific Membrane Slides 1.0 (PEN NF, Zeiss, Oberkochen, Germany). Tumor slides were stained with Arcturus HistoGene LCM Frozen Section Staining Kit (Applied Biosystems, USA) according to manufacturer's instruction. Laser capture microdissection was performed on LSM Palm and tumor tissue was lifted up onto RNAse-free AdhesiveCap 500 opaque (Zeiss, Oberkochen, Germany). RNA was then extracted using Arcturus PicoPure RNA Isolation Kit (Applied Biosystems, USA). After laser microdissection, RNA was first pre-amplified using RT² PreAMP cDNA Synthesis Kit and analysed on RT² Profiler PCR Array according to manufacturer's instruction (Sabioscience, Qiagen, USA). Quantitative RT-PCR was performed on ABI ViiA 7 real time PCR-System (Applied Biosystems, USA).

For phospho-protein analysis, whole slides were lysed with Pathscan Sandwich Elisa Lysis Buffer freshly supplemented with Protease Inhibitor Cocktail (Cell Signaling Technology, USA). Protein lysates were loaded onto PathScan Akt Signaling Antibody Array Kit (Cell Signaling Technology, USA). Protein array near infrared detection were done on Odissey CLx (Li-cor, USA).

In the phospho-protein assay, the pAkt/mTOR/pS6/p4EBP axis was significantly activated in podoplanin-expressing invading tumor cells in comparison to podoplanin-negative tumor cells (Figure 1). In the mRNA array, podoplanin-positive tumors have a significant activation of non-canonical Wnt pathway (WNT5a, BMP7), whereas canonical Wnt pathway was downregulated (CTNNB1, TCF4), see Table 2. This is a surrogate marker of activation of PI3K/mTOR signaling.

**Table 2**

| Gene symbol | Fold change | *P*-value |
|---|---|---|
| WNT5a | 6.9 | 0.008 |
| BMP7 | 4.6 | 0.0036 |
| TCF4 | -2.2 | 0.018 |
| CTNNB1 | -2.6 | 0.014 |

| | | |
|---|---|---|
| WNT5a = WNT5A BMP7 = Bone morphogenetic protein 7 TCF4 = Transcription factor 4 CTNNB1 = Catenin beta-1 | | |

The results are additional evidence for PI3K/mTOR activation based on preclinical data indicating that non-canonical Wnt pathway activates the Pi3K-AKT-mTOR axis (M. Bordonaro et al., PIoS one. 2011, 6(11); M. Almeida et al., J Biol Chem. 2005 280: 41342-41351). Thus, both assays corroborate the finding that podoplanin-expressing tumor cells have activated PI3K/mTOR signaling. Of note, no significant EGFR-Erk 1/2 activation was detected, underlining the specificity of PI3K/mTOR activation in podoplanin-expressing cells. PI3K/mTOR signaling was more strongly correlated with podoplanin-expression than with the histological type of the tumor: while podoplanin-positve squamous cell cancers feature a robust PI3K/mTor activation, podoplanin-negative squamous cell carcinomas have a similarly low PI3K/mTOR activation level as adenocarcinomas of the lung (which are usually podoplanin-negative as well).

### Example

A biopsy of human tumor tissue is performed. The tissue is immediately frozen at -80 C°, shortly fixed in paraformaldehyde and embedded in OCT (optimal temperature cutting) medium. The tissue block is then cut in slices, which are fixed on a slide. Staining for podoplanin is performed on the slide with a primary antibody against podoplanin and a secondary antibody which is labeled with a fluorescent or immunohistochemical dye. Podoplanin-positivity indicates activation of PI3K and/or mTOR signaling. In this case, a therapeutic approach against PI3K and/or mTOR signaling should be considered.

## Claims

1. A method of determining whether, in a malignant human tumor, the PI3K and/or mTOR signaling pathway is activated, **characterized in that** tumor cells are analysed for the upregulation of the basal level of podoplanin, and the malignant tumor is classified as using the PI3K and/or mTOR signaling pathway if podoplanin is present in a higher amount than the basal level.

2. The method of claim 1 wherein the tumor cells are analysed by anti-podoplanin monoclonal antibody.

3. The method of claim 2 wherein anti-podoplanin antibodies are detected by a secondary antibody carrying a fluorescent dye marker or a marker for immunohistochemistry.

4. Podoplanin as a biomarker for activated PI3K/mTOR signalling in human malignant tumors.

5. A method of treating a malignant tumor in a human patient, wherein the upregulation of the basal level of podoplanin in the tumor is determined, and, if podoplanin is present in a higher amount than the basal level, a drug active against PI3K and/or mTOR signaling pathways is administered to said human patient.


SEQUENCE LISTING

<110>  Universitätsspital Basel

<120>  Podpoplanin as biomarker of the activation of PI3K/mTOR signaling
        in human tumors

<130>  P3149EP00

<160>  4

<170>  PatentIn version 3.5

<210>  1
<211>  20
<212>  DNA
<213>  Artificial

<220>
<223>  Synthetic construct

<400>  1
aaatgtcggg aaggtactcg                                                    20


<210>  2
<211>  20
<212>  DNA
<213>  Artificial

<220>
<223>  Synthetic construct

<400>  2
agggcacaga gtcagaaacg                                                    20


<210>  3
<211>  24
<212>  DNA
<213>  Artificial

<220>
<223>  Synthetic construct

<400>  3
tgaccttgat ttattttgca tacc                                               24


<210>  4
<211>  20
<212>  DNA
<213>  Artificial

<220>
<223>  Synthetic construct

<400>  4
cgagcaagac gttcagtcct                                                    20